# EUROPÄISCHE PATENTSCHRIFT

(11) **EP 1 197 185 B1**
(45) Veröffentlichungstag und Bekanntmachung des Hinweises auf die Patenterteilung: **14.07.2004**
(21) Anmeldenummer: 00121374.3
(22) Anmeldetag: 11.10.2000
(51) Int. Cl.: A61B 19/00

(54) **Vorrichtung zum Erfassen oder Verfolgen der Position eines Knochens**
Device for determining or tracking the position of a bone
Dispositif pour déterminer ou suivre la position d'un os

(43) Veröffentlichungstag der Anmeldung: 17.04.2002
(73) Patentinhaber: Stryker Leibinger GmbH & Co. KG, 79111 Freiburg (DE)
(72) Erfinder: Moctezuma de la Barrera, José, Dr.-Ing., 79104 Freiburg (DE); Böhringer, Markus J., Dipl.-Ing., 79238 Ehrenkirchen (DE)
(74) Vertreter: von Hellfeld, Axel, Dr. Dipl.-Phys.

(56) Entgegenhaltungen:
- WO-A-96/11624
- WO-A-99/15097
- US-A- 2 631 585

## Beschreibung

Die Erfindung betrifft eine Vorrichtung zuz Halterung eines Positionsgebers an einem Knochen gemäß dem Oberbegriff des Anspruches 1. Die Erfindung betrifft weiterhin ein System, welches diese Vorrichtung enthält.

Bei medizinischen Operationen ist es unter Umständen erforderlich, vor der Operation beispielsweise durch ein Computertomogramm oder ein Kernspintomogramm Daten über die internen Körperstrukturen zu gewinnen und diese dann bei der Operation zum Beispiel auf einem Monitor dem operierenden Arzt zur Verfügung zu stellen. Problematisch ist, daß sich während der Operation die internen Körperstrukturen etwa durch Atembewegungen des Patienten oder durch den chirurgischen Eingriff des Arztes verschieben können. Eine Verschiebung eines Knochens bedeutet jedoch, daß die zuvor aufgenommenen Bilder nicht mehr die tatsächliche Lage des Knochens wiedergeben und der Arzt dadurch bei einem chirurgischen Eingriff möglicherweise den falschen Zugang plant.

Es sind Vorrichtungen bekannt, welche eine Bewegung eines Körperteiles messen und die Momentanposition des Körperteils bestimmen und verfolgen. Diese Vorrichtungen arbeiten mit Positionsgebern in Form von beispielsweise Strahlungssendern, welche an Halterahmen befestigt werden. Die Halterahmen wiederum werden an dem Körperteil festgelegt. In der Praxis hat sich herausgestellt, daß die Befestigung von Positionsgebern an derartigen Rahmen zwar eine Ortsbestimmung des Rahmens ermöglicht und damit auch eine Ortsbestimmung des im Rahmen gehaltenen Körperteils. Die Genauigkeit einer solchen Ortsbestimmung ist jedoch verbesserungsfähig, da die Halterahmen nicht direkt am Knochen befestigt werden, sondern beispielsweise am Kopf oder am Arm. Außerdem sind geringfügige Verschiebungen zwischen dem Körperteil und dem Rahmen nicht mit Sicherheit auszuschließen.

Um die mit derartigen Rahmen verbundenen Probleme zu vermeiden, wird in der DE 295 03 001 vorgeschlagen, auf den Rahmen zu verzichten und stattdessen die Positionsgeber mit Hilfe von Knochenschrauben direkt am Knochen zu fixieren. Die Knochenschrauben weisen dabei einen Schraubenkörper auf, welcher als Aufnahme für den Positionsgeber fungiert.

Auch die aus der DE 295 03 001 bekannte Vorrichtung zur Erfassung bzw. Verfolgung der Position eines Knochens ist jedoch mit einer Reihe von Nachteilen behaftet. So ist sowohl das Befestigen als auch das Lösen der Knochenschrauben mit zeitlichem und apparativem Aufwand verbunden. Besonders ausgeprägt ist diese Problematik, wenn die Schrauben im Körperinneren des Patienten plaziert werden müssen. Außerdem ist es bei einer Vielzahl von Anwendungen aufgrund der durch das Einschrauben hervorgerufenen Beeinträchtigung des Knochens nicht möglich, Knochenschrauben als Aufnahme für die Positionsgeber zu verwenden. Eine Verwendung der aus der DE 295 03 001 bekannten Vorrichtung im Körperinneren des Patienten scheidet auch häufig deswegen aus, weil die von den Knochenschrauben aufgenommenen Positionsgeber in Form von Leuchtdioden während der Operation nicht oder nur schlecht sichtbar sind. Die aus der DE 295 03 001 bekannte Vorrichtung eignet sich deswegen praktisch nur für die Erfassung und Verfolgung der Position eines Schädelknochens.

Aus WO 96/11624 und US 2,631,585 sind zudem Knochenklemmen mit einem Paar schwenkbarer Klemmbacken bekannt, welche von entgegengesetzten Seiten her den Knochen zwischen sich einklemmen.

Der Erfindung liegt die Aufgabe zugrunde, eine Vorrichtung zur Halterung eines Positionsgebers an einem Knochen zur Verfügung zu stellen, welche einfach, aber dennoch zuverlässig an einem Knochen, insbesondere im Körperinneren, befestigt und von diesem wieder gelöst werden kann. Weiterhin soll die Vorrichtung einer Ausgestaltung zugänglich sein, welche die Erfassung der vom Positionsgeber bereitgestellten Signale vor allem dann vereinfacht, wenn die Vorrichtung an einem sich im Körperinnern befindenden Knochen befestigt wird. Eine weitere Aufgabe ist es schließlich, ein die erfindungsgemäße Vorrichtung enthaltendes System zur Erfassung und/oder Verfolgung der Position eines Knochens anzugeben.

Diese Aufgaben werden gelöst durch eine Vorrichtung gemäß Anspruch 1 und ein System gemäß Anspruch 10. Die Unteransprüche betreffen vorteilhafte Ausgestaltungen und Weiterbildungen der Erfindung.

Eine erfindungsgemäße Vorrichtung zur Halterung eines Positionsgebers an einem Knochen umfasst einen Grundkörper mit einer Aufnahmehalterung für den Positionsgeber sowie einen Greifmechanismus zur Befestigung des Grundkörpers am Knochen. Der Greifmechanismus umfasst mindestens zwei schwenkbare Greifelemente, welche in einer Greifstellung des Greifmechanismus von verschiedenen Seiten her am Knochen angreifen. Femer weist der Greifmechanismus eine Betätigungseinrichtung für die Greifelemente auf.

Erfindungsgemäß umfasst der Greifmechanismus mindestens ein weiteres Greifelement, welches in der Greifstellung des Greifmechanismus von einer weiteren Seite her am Knochen angreift. Die schwenkbaren Greifelemente sind dabei durch die Betätigungseinrichtung derart relativ zu dem mindestens einen weiteren Greifelement bewegbar, dass im Zuge des Umschließens des Knochens durch die schwenkbaren Greifelemente eine relative Annäherungsbewegung zwischen dem Knochen und dem mindestens einen weiteren Greifelement bewirkt wird. Der Greifmechanismus bewirkt demnach als Folge des Umschließens des Knochens durch die schwenkbaren Greif elemente eine erzwungene Relativbewegung zwischen dem Knochen und dem weiteren Greifelement, die letztendlich dazu führt, dass der Knochen gegen das weitere Greifelement gepresst wird. Die Greifelemente der erfindungsgemäßen Vorrichtung wirken in ihrer Gesamtheit so zusammen, dass der Knochen von drei oder mehr Seiten her umschlossen wird. Die so erzielte Drei- oder Mehrpunktauflage gewährleistet einen optimalen Krafttluss. Vorteilhafterweise gestattet es die Betätigungseinrichtung, die schwenkbaren Greifelemente in der Greifstellung zu arretieren.

Besonders bevorzugt findet das Zusammenwirken zwischen einem schwenkbaren Greifelement und der Betätigungseinrichtung derart statt, daß ein zumindest bereichsweise keilförmiges Element auf ein schwenkbares Greifelement oder gleichzeitig auf mehrere schwenkbare Greifelemente einwirkt. Auf diese Weise können durch Bewegen des Keilelementes die schwenkbaren Greifelemente betätigt werden. Auch ist es denkbar, das Keilelement dazu zu verwenden, die schwenkbaren Greifelemente in einer den Knochen umschließenden Lage mit einer vorgegebenen Klemmspannung zu arretieren, um dadurch die Aufnahme für den Positionsgeber fest mit dem Knochen zu verbinden. Somit wird eine einfache aber zuverlässige Verbindung zwischen dem Knochen und der erfindungemäßen Halterungsvorrichtung gewährleistet.

In einer bevorzugten Ausgestaltung des Greifmechanismus ist jedes schwenkbare Greifelement als Hebelarm ausgestaltet, welcher Teil eines drehbar gelagerten Hebels ist. Der Betätigungsmechanismus kann dann beispielsweise mittels des Keilelements mit diesem Hebelarm oder mit einem oder mehreren weiteren Hebelarmen des Hebels zusammenwirken

Eine weitere Ausgestaltung der Vorrichtung sieht vor, daß der Grundkörper einen langgestreckten Abschnitt aufweist, welcher die Greifelemente von einem Bedienungselement der Betätigungseinrichtung räumlich trennt. So ist es denkbar, im Bereich des einen Endes des langgestreckten Abschnittes die Greifelemente anzuordnen und im Bereich des gegenüberliegenden Endes das Bedienungselement für die Betätigungseinrichtung vorzusehen. Eine derartige Ausgestaltung des Grundkörpers erlaubt es, das Bedienungselement räumlich weit beabstandet vom Knochen anzuordnen. Dies ist vor allem bei chirurgischen Eingriffen zweckmäßig, da durch eine ausreichende Länge des langgestreckten Abschnittes das Bedienungselement aus dem Körper des Patienten herausgeführt werden kann. Aus dem gleichen Grund ist es vorteilhaft, den Positionsgeber ebenfalls im Bereich des Bedienungselementes, d. h. an dem den Greifelementen gegenüberliegenden Ende des Grundkörpers zu befestigen.

Weitere Vorteile und Ausgestaltungen der Erfindung ergeben sich aus dem Ausführungsbeispiel und den Figuren. Es zeigen:
- Figur 1: eine Seitenansicht der erfindungsgemäßen Vorrichtung,
- Figur 2: die Aufnahme für einen Positionsgeber im Querschnitt,
- Figur 3: ein Greifelement in Aufsicht und in Querschnitten,
- Figur 4: den Betätigungsmechanismus für die Greifelemente in einer den Knochen umschließenden Position,
- Figur 5: den Betätigungsmechanismus für die Greifelemente in geöffneter Position,
- Figuren 6 und 7: die Befestigung der erfindungsgemäßen Vorrichtung auf dem Dornfortsatz eines Wirbelkörpers, und
- Figur 8: ein System zum Erfassen oder Verfolgen der Position eines Knochens.

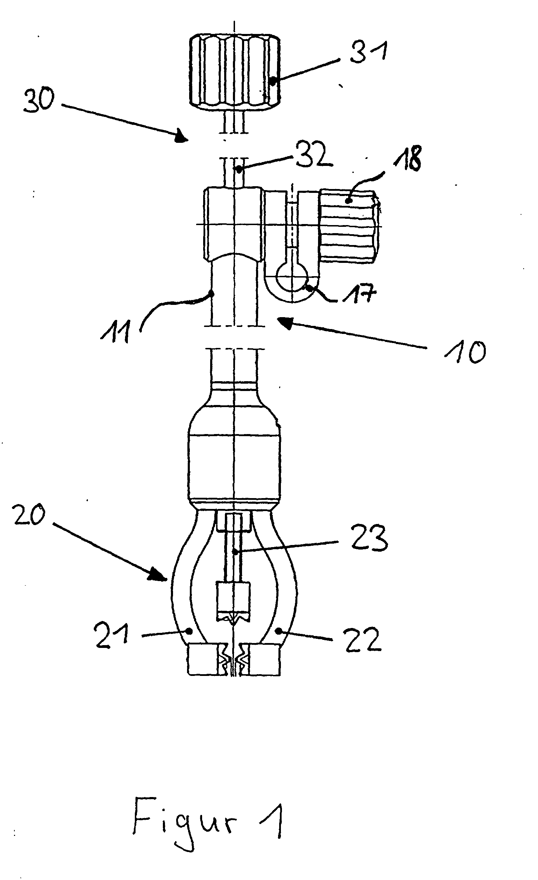

In Figur 1 ist ein Ausführungsbeispiel einer Vorrichtung zur Halterung eines Positionsgebers an einem Knochen in einer Seitenansicht dargestellt. Die Vorrichtung umfaßt eine auch als Grundkörper bezeichnete Aufnahme 10 für einen Positionsgeber sowie einen Greifmechanismus 20, 30. Der Greifmechanismus umfaßt eine Gesamtheit von Greifelementen 20 sowie eine Betätigungseinrichtung 30 für einen Schwenkmechanismus, welcher zwei schwenkbare Greifelemente 21, 22 umfaßt. Die Betätigungseinrichtung 30 wiederum weist ein Bedienungselement in Form eines Drehgriffes 31 auf. Der Drehgriff 31 ist drehfest mit einer Welle 32 der Betätigungseinrichtung 30 verbunden.

Bei dem in Figur 1 dargestellten Ausführungsbeispiel wird die Gesamtheit 20 der Greifelemente von drei Greifelementen 21, 22 und 23 gebildet. Die beiden schwenkbaren Greifelemente 21, 22 sind jeweils als Teil eines Hebels ausgestaltet, welcher mit der Betätigungseinrichtung 30 zusammenwirkt. Das dritte Greifelement 23 ist fest mit der Aufnahme 10 verbunden. Durch die drei Greifelemente 21, 22 und 23 kann ein Knochen auf drei Seiten umschlossen werden.

Wie Figur 1 entnommen werden kann, weist die Aufnahme 10 eine langgestreckte Bauform auf. An dem einen Ende der Aufnahme 10 ist der Drehgriff 31 der Betätigungseinrichtung 30 angeordnet und die Gesamtheit 20 der Greifelemente befindet sich im Bereich des gegenüberliegenden Endes der Aufnahme 10.

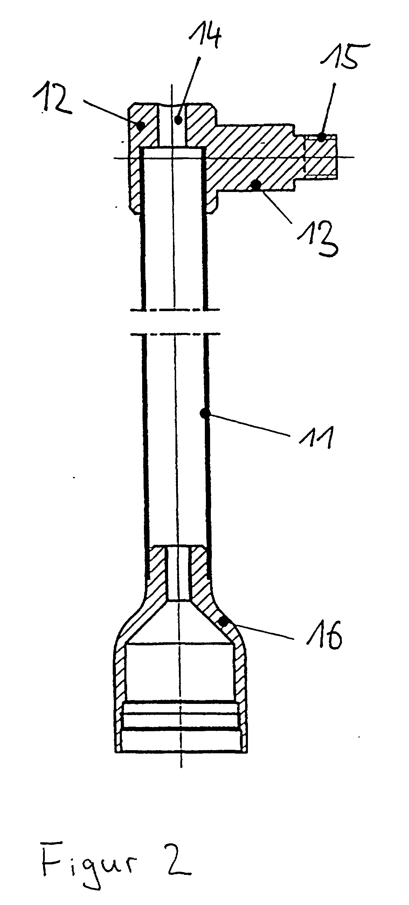

Die Aufnahme 10 ist in Figur 2 im Querschnitt dargestellt. Die Aufnahme 10 umfaßt einen langgestreckten Abschnitt in Form einer als Hohlzylinder ausgestalteten Hülse 11. Die Länge der Hülse 11 bestimmt im wesentlichen den Abstand zwischen dem Drehgriff 31 und den Greifelementen 21, 22 und 23.

Auf dem oberen Ende der Hülse 11 ist ein Aufsatz 12 angeordnet. Dieser Aufsatz 12 umfaßt einen sich in radialer Richtung bezüglich der Hülse 11 erstreckenden zylindrischen Fortsatz 13 zum Befestigen eines Positionsgebers. Am Ende des zylindrischen Fortsatzes 13 ist ein weiterer zylindrischer Abschnitt 15 mit geringerem Durchmesser angeordnet, welcher mit einem Gewinde versehen ist. Der Aufsatz 12 weist weiterhin eine zentrale Bohrung 14 auf, durch welche sich, wie in Figur 1 dargestellt, die Welle 32 erstreckt.

An einem dem Aufsatz 12 gegenüberliegenden Ende der Hülse 11 ist die Hülse 11 mit einem Gehäuse 16 verbunden. Das Gehäuse 16 weist im wesentlichen die Form eines Hohlzylinders mit größerem Querschnitt als die Hülse 11 auf. Das Innere des Gehäuses 16 besitzt einen in Richtung auf die Hülse 11 sich konisch verjüngenden Abschnitt.

Aufsatz 12, Hülse 11 und Gehäuse 16 sind durch Laserschweißen miteinander verbunden. Es ist jedoch auch denkbar, den Aufsatz 12 derart mit der Hülse 11 zu verbinden, daß der Aufsatz 12 um die Längsachse der Hülse 11 schwenkbar ist. Weiterhin können Hülse 11, Aufsatz 12 und Gehäuse 16 auch einstückig ausgestaltet werden.

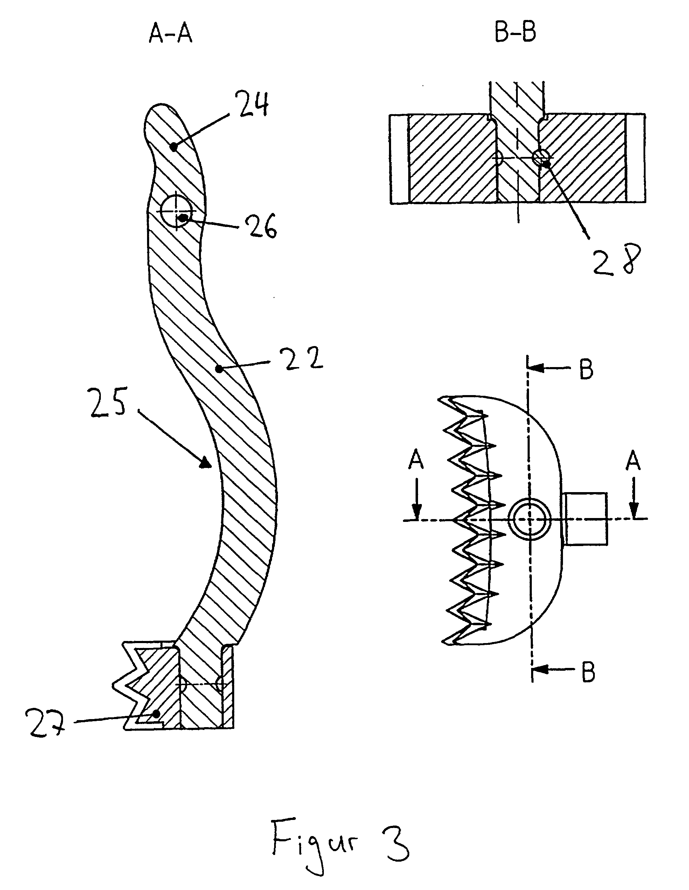

In Figur 3 ist die Ausgestaltung des schwenkbaren Greifelementes 22 in Aufsicht sowie in zwei Schnitten dargestellt. Gemäß Figur 3 ist das bewegliche Greifelement 22 als Teil eines Hebels 25 ausgebildet. Der Hebel 25 besitzt einen geschwungenen, das Greifelement 22 bildenden ersten Hebelarm, an dessen Ende ein Backen 27 angeordnet ist (Schnitt A-A). Der Hebel 25 umfaßt weiterhin einen zweiten Hebelarm 24, welcher mit der Betätigungseinrichtung für die Greifelemente zusammenwirkt. Zwischen dem ersten Hebelarm 22 und dem zweiten Hebelarm 24 ist eine Bohrung 26 zum Lagern des Hebels 25 angeordnet.

Der Backen 27 ist mit Zähnen ausgestattet, welche eine zuverlässige Verbindung zwischen dem Greifelement 22 und dem Knochen gewährleisten. Zur Unterstützung dieses Zweckes ist der Backen 27 um die Hebellängsachse drehbar ausgestaltet. Wie dem Schnitt B-B entnommen werden kann, wird der Backen 27 mit Hilfe einer Schraube 28 drehbar mit dem Hebel 22 verbunden.

Gemäß einer alternativen Ausführungsform kann der Backen 27 jedoch auch drehfest mit dem Hebel 22 verbunden sein.

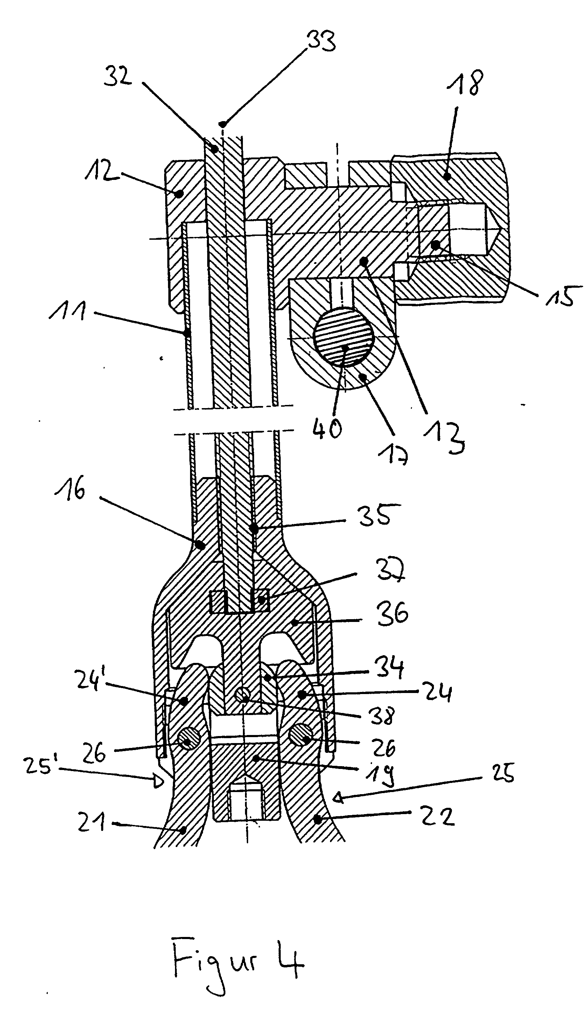

In Figur 4 ist eine Möglichkeit zur Befestigung eines Positionsgebers 40 an der Aufnahme 11 dargestellt. Der Positionsgeber 40 ist über eine Schelle 17, welche auf dem zylindrischen Fortsatz 13 des Aufsatzes 12 sitzt, mit der Aufnahme 11 verbunden. Mit Hilfe einer Feststelleinrichtung für die Schelle 17 in Form einer Mutter 18, welche mit dem Gewinde des zylindrischen Abschnittes 15 zusammenwirkt, kann die Schelle 17 betätigt werden. Durch Bewegen der Mutter 18 in Richtung auf die Hülse 11 wird der Positionsgeber 40 fest von der Schelle 17 umschlossen. Durch Bewegen der Mutter 18 in die entgegengesetzte Richtung wird der Positionsgeber 40 wieder freigegeben.

In Figur 4 ist weiterhin die Position und Funktion der Betätigungseinrichtung skizziert, wenn die schwenkbaren Greifelemente 21, 22 einen Knochen umgreifen. Die Betätigungseinrichtung umfaßt ein keilförmiges Element 34, welches mit einer Welle 32 gekoppelt ist. Die Welle 32 wiederum ist in einem Gewinde 35, welches im Bereich eines an die Hülse 11 angrenzenden Halses des Gehäuses 16 ausgebildet ist, geführt. Durch Drehen der Welle 32 um die Wellenlängsachse 33 kann das Keilelement 34 axial zur Wellenlängsachse 33 verschoben werden.

Das Keilelement 34 wirkt mit den beiden zweiten Hebelarmen 24, 24' der beiden Hebel 25, 25' zusammen. Das Keilelement 34 greift dazu in eine Ausbuchtung der zweiten Hebelarme 24, 24' ein. Durch den Eingriff in die Ausbuchtung der beiden Hebelarme 24, 24' wird bewirkt, daß durch ein Verschieben des Keilelementes 34 nach oben, d. h. in Richtung auf den Aufsatz 12, die Enden der beiden Hebelarme 24, 24' voneinander weg bewegt werden. Dieses Auseinanderdrücken der Enden der Hebelarme 24, 24' durch das Keilelement 34 wiederum bewirkt, daß die Backen der schwenkbaren Greifelemente 21, 22 aufeinander zu bewegt werden, um einen Knochen zu umschließen. Durch das Zusammenwirken von Gewinde 35, Welle 32 und dem die zweiten Hebelarme 24, 24' kontaktierenden Keilelement 34 wird erreicht, daß die schwenkbaren Greifelemente 21, 22 in einer den Knochen fest umschließenden Position arretierbar sind.

Das Keilelement 34 ist über ein sich in Richtung auf den Aufsatz 12 konisch verjüngendes Bauteil 36 mit der Welle 32 gekoppelt. Zu diesem Zweck besitzt das konische Bauteil 36 einen sich entlang der Wellenlängsachse 33 in Richtung auf die Greifelemente 21, 22, 23 erstreckenden zapfenförmigen Fortsatz, mit welchem das ringförmige Keilelement 34 durch einen Stift 38 verbunden ist. Die Welle 32 weist an einem dem Drehgriff gegenüberliegenden Ende einen fest mit der Welle 32 verbundenen Ring 37 auf. Dieser Ring 37 wiederum ist drehbar in einer Aussparung des konischen Bauteiles 36 gelagert. Auf diese Weise wird die Drehbewegung der Welle 32 von der Bewegung des konischen Bauteiles 36 und des Keilelementes 34 axial zur Wellenlängsachse 33 entkoppelt. Die Drehbewegung der Welle 32 überträgt sich daher nicht auf das konische Bauteil 36 und das Keilelement 34. Obwohl die geschilderte Verbindung zwischen Welle 32, konischem Bauteil 36 und Keilelement 34 bevorzugt ist, könnte grundsätzlich auch daran gedacht werden, diese drei Elemente einstückig auszuführen.

Bei einer axialen Bewegung des konischen Bauteiles 36 kann dieses mit der konischen Aussparung des Gehäuses 16 zusammenwirken. Auf diese Weise wird eine Selbstzentrierung der Betätigungseinrichtung erreicht.

Das Gehäuse 16 ist auf seiner der Hülse 11 gegenüberliegenden Seite durch einen Deckel 19 verschlossen. Gemäß dem Ausführungsbeispiel in Figur 4 ist zwischen dem Gehäuse 16 und dem Deckel 19 eine Schraubverbindung vorgesehen. Die Hebel 25, 25' sind im Deckel 19 drehbar gelagert. Dies geschieht mit Hilfe von Stiften, welche in die Bohrungen 26 der Hebel 25, 25' eingeführt werden.

Der Deckel 19 besitzt einen sich entlang der Wellenlängsachse 33 in Richtung auf den Knochen erstreckenden zapfenförmigen Fortsatz. Dieser Fortsatz ist mit einer ein Innengewinde aufweisenden Sacklochbohrung versehen, welche als Aufnahme für ein feststehendes Greifelement fungiert. Dieser Sachverhalt ist für das feststehende Greifelement 23 in Figur 1 dargestellt. In einer alternativen Ausgestaltung der in Figur 4 dargestellten Vorrichtung sind entweder die Aufnahme für das feststehende Greifelement 23 oder das feststehende Greifelement 23 oder beide höhenverstellbar. Dies bedeutet, daß die Aufnahme oder das Greifelement 23 oder beide axial zur Wellenlängsachse 33 beweglich sind.

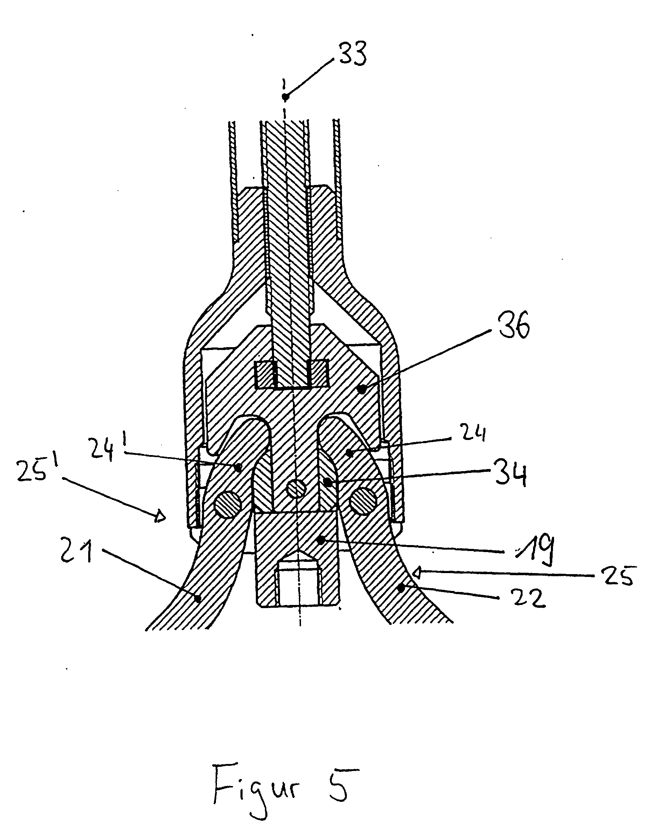

In Figur 5 ist die Betätigungseinrichtung in einem geöffneten Zustand dargestellt. Sobald das Keilelement 34 durch Drehen der Welle 32 in Richtung auf den Deckel 19 bewegt wird, werden die Enden der zweiten Hebelarme 24, 24' nicht länger auseinandergedrückt und dadurch werden auch die Enden der ersten Hebelarme 25, 25' nicht länger gegeneinander gedrückt. In dieser Position des Keilelementes 34 wird der von den Greifelementen umschlossene Knochen daher wieder freigegeben.

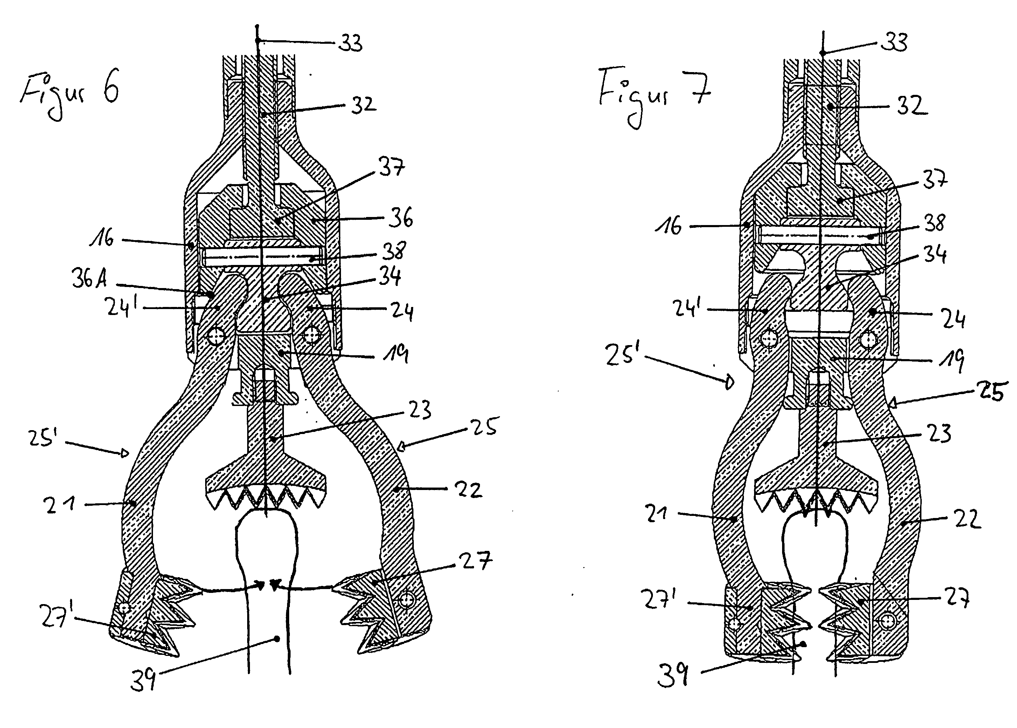

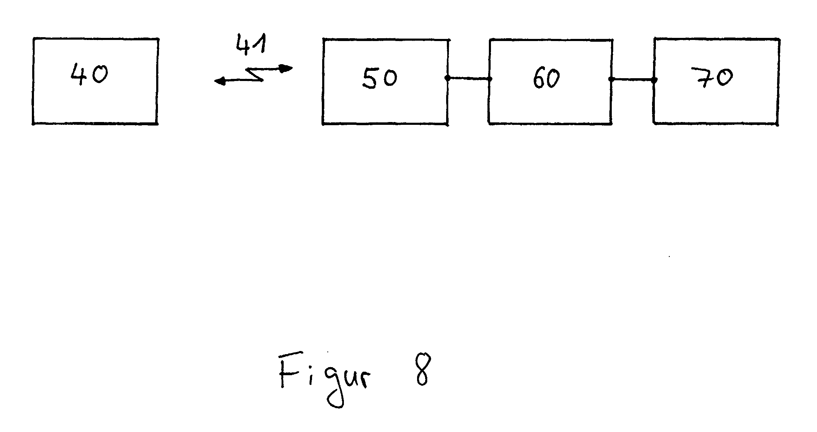

In den Figuren 6 und 7 ist beispielhaft die Befestigung der erfindungsgemäßen Vorrichtung auf dem Dornfortsatz 39 eines Wirbelkörpers dargestellt. Die in den Figuren 6 und 7 dargestellte Vorrichtung weicht in der Kopplung des Keilelements 34 mit der Welle 32 von der in den Figuren 4 und 5 dargestellten Vorrichtung ab. Anders als bei der in den Figuren 4 und 5 dargestellten Vorrichtung ist das Keilelement 34 nicht als Ring ausgebildet, welcher auf einem sich in Richtung auf den Knochen erstreckenden zapfenförmigen Fortsatz des konischen Bauteils 36 angeordnet ist, sondern als massives Bauteil. Weiterhin ist bei der in den Figuren 6 und 7 dargestellten Vorrichtung der in einer Aussparung des konischen Bauteils 36 gelagerte Ring 37 einstückig mit der Welle 32 ausgebildet.

In Figur 6 ist die Vorrichtung in geöffneter Stellung um einen Dornfortsatz 39 eines Wirbelkörpers plaziert. Durch Drehen der Welle 32 wurde das Keilelement 34 in Anlage mit dem Deckel 19 gebracht. Das Keilelement 34 und das konische Bauteil 36 befinden sich damit bezüglich des Gehäuses 16 in ihrer untersten Stellung. Beim Bewegen in diese Stellung wirkt der radial innere Rand 36A einer dem Dornfortsatz 39 zugewandten ringförmigen Stirnfläche des konischen Bauteils 36 mit den zweiten Hebelarmen 24, 24' der beiden Hebel 25, 25' derart zusammen, daß die dem Dornfortsatz 39 abgewandten Enden der zweiten Hebelarme 24, 24' bezüglich der Längsachse 33 radial nach innen verschoben werden. Folglich werden diejenigen Enden der beiden schwenkbaren Greifelemente 21, 22, welche die Backen 27, 27' tragen, radial nach außen bewegt. In dieser Stellung wird die Vorrichtung nun derart auf den Dornfortsatz 39 aufgesetzt, daß die Zähne des feststehenden Greifelementes 23 die der Vorrichtung zugewandte Oberseite des Dornfortsatzes 39 kontaktieren.

Wird aus der in Figur 6 dargestellten Stellung heraus das konische Bauteil 36 und das mit dem konischen Bauteil 36 gekoppelte Keilelement 34 durch Drehen der Welle 32 bezüglich des Gehäuses 16 nach oben verschoben, so wirkt das Keilelement 34 mit den beiden dem Dornfortsatz 39 abgewandten Enden der zweiten Hebelarme 24, 24' derart zusammen, daß diese Enden bezüglich der Längsachse 33 radial nach außen bewegt werden. Folglich führen die beiden schwenkbaren Greifelemente 21, 22, welche jeweils die ersten Hebelarme der Hebel 25, 25' bilden, eine Schwenkbewegung in Richtung auf den Dornfortsatz 39 aus und nähern sich diesem, wie in Figur 7 dargestellt, von zwei gegenüberliegenden Seiten. Aufgrund dieser Schwenkbewegung der beiden schwenkbaren Greifelemente 21, 22 findet eine Zwangsbewegung des feststehenden Greifelements 23 in Richtung auf eine dritte Seite des Dornfortsatzes 39 statt.

Diese Zwangsbewegung ist darauf zurückzuführen, daß sich die beiden schwenkbaren Greifelemente 21, 22 dem Dornfortsatz 39 auf einer geschwungenen Bahn nähern. Infolge der geschwungenen Bahn kontaktiert zunächst die obere Zahnreihe jedes der Backen 27, 27' den Dornfortsatz 39. Kommen die oberen Zahnreihen nun in Kontakt mit dem Dornfortsatz 39, bewirkt ein weiteres Bewegen der zweiten Hebelarme 24, 24' radial nach außen eine Zugbewegung des feststehenden Greifelementes 23 in Richtung auf die diesem Greifelement 23 zugewandte Oberfläche des Dornfortsatzes 39. Diese Zugbewegung endet erst, wenn alle Zahnreihen der Backen 27, 27' den Dornfortsatz 39 kontaktieren. Die den Dornfortsatz 39 zugewandten Zähne des feststehenden Greifelements 23 dringen daher geringfügig in den Dornfortsatz 39 ein. Die Vorrichtung umschließt den Fortsatz 39 folglich zuverlässig auf drei Seiten, wobei aufgrund des Eindringens der Zähne des feststehenden Greifelements 23 in den Dornfortsatz 39 eine besonders feste Verbindung gewährleistet ist.

Bevorzugt kommt die beschriebene Vorrichtung in einem System zum Erfassen oder Verfolgen der Position eines Knochens zum Einsatz. Ein solches System ist schematisch in Figur 8 dargestellt. Bei diesem System wird in der Aufnahme der Vorrichtung ein Positionsgeber 40, beispielsweise eine elektromagnetische Strahlung 41 aussendende Strahlungsquelle, angeordnet. Bevorzugt kommen Strahlungsquellen zum Einsatz, welche Licht im sichtbaren Wellenlängenbereich aussenden. In Frage kommen beispielsweise Leuchtdioden oder Laserdioden.

Das System umfaßt einen Detektor 50 zur Erfassung eines vom Positionsgeber 40 abgegebenen Signales. Weiterhin ist Teil des Systemes eine Auswerteeinheit 60, welche anhand eines Ausgangssignales des Detektors 50 und eines bereits vorher erstellten Patientenbildes einen Vergleich der aktuellen Lage des Knochens und der Lage des Knochens auf dem Patientenbild durchführt. Von der Auswerteeinheit 60 kann dann ein neues Patientenbild generiert werden, welches den Knochen in seiner aktuellen Position zeigt. Über einen Monitor oder eine andere Anzeigeeinheit 70 wird dieses Bild dann dem operierenden Arzt zur Verfügung gestellt.

## Patentansprüche

1. Vorrichtung zur Halterung eines Positionsgebers (40) an einem Knochen (39), umfassend
- einen Grundkörper (11, 12, 16) mit einer Aufnahmehalterung (12) für den Positionsgeber (40) sowie
- einen Greifmechanismus (20, 30) zur Befestigung des Grundkörpers (11, 12, 16) am Knochen (39), wobei der Greifmechanismus (20, 30) mindestens zwei schwenkbare Greifelemente (21, 22) umfasst, welche in einer Greifstellung des Greifmechanismus von verschiedenen Seiten her am Knochen (39) angreifen, wobei der Greifmechanismus (20, 30) ferner eine Betätigungseinrichtung (30) für die Greifelemente (21, 22) umfasst,
**dadurch gekennzeichnet, dass** der Greifmechanismus (20, 30) mindestens ein weiteres Greifelement (23) umfasst, welches in der Greifstellung des Greifmechanismus von einer weiteren Seite her am Knochen (39) angreift, wobei die schwenkbaren Greifelemente (21, 22) durch die Betätigungseinrichtung (30) derart relativ zu dem mindestens einen weiteren Greifelement (23) bewegbar sind, dass im Zuge des Umschließens des Knochens (39) durch die schwenkbaren Greifelemente (21, 22) eine relative Annäherungsbewegung zwischen dem Knochen (39) und dem mindestens einen weiteren Greifelement (23) bewirkt wird.

2. Vorrichtung nach Anspruch 1,
**dadurch gekennzeichnet, dass** insgesamt zwei schwenkbare Greifelemente (21, 22) vorgesehen sind, die in der Greifstellung des Greifmechanismus (20, 30) von gegenüberliegenden Seiten her am Knochen (39) angreifen.

3. Vorrichtung nach Anspruch 1 oder 2,
**dadurch gekennzeichnet, dass** die schwenkbaren Greifelemente (21, 22) jeweils als erster Hebelarm eines schwenkbar an dem Grundkörper (11, 12, 16) gelagerten Hebels (25, 25') ausgebildet sind und dass die Betätigungseinrichtung (30) an dem ersten oder einem zweiten (24, 24') Hebelarm jedes Hebels (25, 25') angreift.

4. Vorrichtung nach Anspruch 3,
**dadurch gekennzeichnet, dass** jeder erste Hebelarm einen Backen (27, 27') mit mehreren Zahnreihen trägt, die im Zuge des Umschließens des Knochens (39) nacheinander in Eingriff mit dem Knochen (39) gelangen.

5. Vorrichtung nach Anspruch 4,
**dadurch gekennzeichnet, dass** die Backen (27, 27') an ihrem jeweiligen ersten Hebelarm (21, 22) relativ zu diesem um eine Hebellängsachse drehbar, jedoch feststellbar angeordnet sind.

6. Vorrichtung nach einem der Ansprüche 1 bis 5,
**dadurch gekennzeichnet, dass** die Betätigungseinrichtung (30) ein Keilelement (34) umfasst, welches zur Betätigung der schwenkbaren Greifelemente (21, 22) geradlinig verstellbar ist.

7. Vorrichtung nach Anspruch 6,
**dadurch gekennzeichnet, dass** das Keilelement (34) mit einem Längsende einer in einem langgestreckten Hülsenabschnitt (11) des Grundkörpers (11, 12, 16) längsverstellbar aufgenommenen Betätigungswelle (32) gekoppelt ist, welche an ihrem entgegengesetzten Längsende aus dem Hülsenabschnitt (11) herausragt und dort ein Handbetätigungselement (31) trägt.

8. Vorrichtung nach einem der Ansprüche 1 bis 7,
**dadurch gekennzeichnet, dass** das mindestens eine weitere Greifelement (23) fest an dem Grundkörper (11, 12, 16) angebracht ist.

9. Vorrichtung nach einem der Ansprüche 1 bis 7,
**dadurch gekennzeichnet, dass** das mindestens eine weitere Greifelement (23) geradlinig verstellbar, jedoch festlegbar an dem Grundkörper (11, 12, 16) gehalten ist.

10. System zum Erfassen oder Verfolgen der Position eines Knochens (39), umfassend eine Vorrichtung nach einem der Ansprüche 1 bis 9, einen an der Aufnahmehalterung (12) des Vorrichtungsgrundkörpers (11, 12, 16) angebrachten Positionsgeber (40), einen Detektor (50) zur Erfassung eines vom Positionsgeber (40) abgegebenen Signals sowie eine an den Detektor (50) angeschlossene Auswerteeinheit (60), welche anhand eines Ausgangssignals des Detektors (50) und eines zuvor erstellten Patientenbilds eine Abweichung der aktuellen Lage des Knochens (39) von der Lage des Knochens auf dem Patientenbild ermittelt.

11. System nach Anspruch 10,
**dadurch gekennzeichnet, dass** von der Auswerteeinheit (60) anhand des zuvor erstellten Patientenbilds und des Detektorausgangssignals ein Patientenbild generierbar ist, welches die aktuelle Position des Knochens (39) zeigt, und
dass weiterhin eine Anzeigeeinheit (70) zum Anzeigen des derart generierten Patientenbilds vorhanden ist.

## Claims

1. A device for holding a position transmitter (40) at a bone (39), comprising
- a base body (11, 12, 16) having a locating holder (12) for the position transmitter (40), as well as
- a gripping mechanism (20, 30) for fastening the base body (11, 12, 16) to the bone (39), wherein the gripping mechanism (20, 30) comprises at least two swivelling gripping elements (21, 22) which in a gripping position of the gripping mechanism are applied to the bone (39) from different sides, and wherein the gripping mechanism (20, 30) further comprises an actuating device (30) for the gripping elements (21, 22),
**characterized in that** the gripping mechanism (20, 30) comprises at least one further gripping element (23) which in the gripping position of the gripping mechanism is applied to the bone (39) from a further side, wherein the swivelling gripping elements (21, 22) are adapted to be moved by the actuating device (30) in such a manner relative to the at least one further gripping element (23) that in the course of the bone (39) being surrounded by the swivelling gripping elements (21, 22) a relative approaching movement between the bone (39) and the at least one further gripping element (23) is effected.

2. The device according to Claim 1,
**characterized in that** a total of two swivelling gripping elements (21, 22) is provided which in the gripping position of the gripping mechanism (20, 30) are applied to the bone (39) from opposite sides.

3. The device according to Claim 1 or 2,
**characterized in that** the swivelling gripping elements (21, 22) are each designed as the first lever arm of a lever (25, 25') that is pivotally mounted to the base body (11, 12, 16), and that the actuating device (30) engages at the first or at a second (24, 24') lever arm of each lever (25, 25').

4. The device according to Claim 3,
**characterized in that** each first lever arm carries a jaw (27, 27') having several rows of teeth which, in the course of surrounding the bone (39), get to engage the bone one after the other.

5. The device according to Claim 4,
**characterized in that** at their respective first lever arm (21, 22) the jaws (27, 27') are arranged so as to be rotatable relative to the same about a longitudinal axis of the lever but so as to be lockable.

6. The device according to one of Claims 1 to 5,
**characterized in that** the actuating device (30) comprises a wedge element (34) that is adjustable linearly for operating the swivelling gripping elements (21, 22).

7. The device according to Claim 6,
**characterized in that** the wedge element (34) is coupled to a longitudinal end of an actuator shaft (32) that is received so as to be adjustable in longitudinal direction in an elongated sleeve portion (11) of the base body (11, 12, 16), said actuator shaft (32) protruding at its opposite longitudinal end from the sleeve portion (11) and there carrying a manually operable element (31).

8. The device according to one of claims 1 to 7,
**characterized in that** the at least one further gripping element (23) is firmly mounted to the base body (11, 12, 16).

9. The device according to one of claims 1 to 7,
**characterized in that** the at lest one further gripping element (23) is held at the base body (11, 12, 16) so as to be adjustable linearly but lockable.

10. A system for detecting or following the position of a bone (39), comprising a device according to one of Claims 1 to 9, a position transmitter (40) arranged on the locating holder (12) of the device base body (11, 12, 16), a detector (50) for detecting a signal emitted by the position transmitter (40), as well as an evaluating unit (60) that is connected to the detector (50) and, on the basis of an output signal from the detector (50) and a previously produced patient image, determines a deviation of the current position of the bone (39) from the position of the bone on the patient image.

11. The system according to Claim 10,
**characterized in that** a patient image showing the current position of the bone (39) can be generated by the evaluating unit (60) on the basis of the previously produced patient image and the output signal of the detector, which shows the current position of the bone (39) and that in addition a display unit (70) for displaying the patient image generated in this manner is provided.

## Revendications

1. Dispositif pour la fixation d'un indicateur de position (40) sur un os (39), comprenant
- un corps de base (11, 12, 16) avec un support (12) pour la fixation de l'indicateur de position (40), ainsi que
- un mécanisme de préhension (20, 30) pour la fixation du corps de base (11, 12, 16) sur l'os (39), lequel mécanisme de préhension (20, 30) comporte au moins deux éléments préhenseurs (21, 22) pivotants qui, lorsque le mécanisme de préhension est en position de prise, sont en prises avec l'os (39) depuis différents côtés, et lequel mécanisme de préhension (20, 30) comporte en outre un dispositif d'actionnement (30) pour les éléments préhenseurs (21, 22),
**caractérisé en ce que** le mécanisme de préhension (20, 30) comporte au moins un élément préhenseur (23) supplémentaire, lequel, lorsque le mécanisme de préhension est en position de prise, est en prise avec l'os (39) depuis un autre coté, les éléments préhenseurs (21, 22) pivotants pouvant, à l'aide du dispositif d'actionnement (30), être actionnés de telle sorte par rapport audit élément préhenseur (23) supplémentaire qu'il est généré un mouvement d'approche relatif entre l'os (39) et l'élément préhenseur (23) supplémentaire lorsque les éléments préhenseurs (21, 22) pivotants viennent en prise avec l'os (39).

2. Dispositif selon la revendication 1,
**caractérisé en ce qu'**il est prévu dans l'ensemble deux éléments préhenseurs (21, 22) pivotants qui, lorsque le mécanisme de préhension (20, 30) est en position de prise, sont en prise avec l'os (39) depuis deux côtés opposés.

3. Dispositif selon la revendication 1 ou 2,
**caractérisé en ce que** les éléments préhenseurs (21, 22) pivotants sont respectivement conçus pour former un premier bras d'un levier (25, 25') monté à pivotement sur le corps de base (11, 12, 16) et **en ce que** le dispositif d'actionnement (30) agit sur le premier ou sur un second (24, 24') bras de chaque levier (25, 25').

4. Dispositif selon la revendication 3,
**caractérisé en ce que** chaque premier bras de levier supporte une mâchoire (27, 27') dotée de plusieurs rangées de dents qui viennent successivement en prise avec l'os (39) pendant le mouvement de mise en position autour de l'os (39).

5. Dispositif selon la revendication 4,
**caractérisé en ce que** les mâchoires (27, 27') sont disposées sur leur premier bras de levier (21, 22) respectif de manière à pouvoir pivoter par rapport audit bras de levier autour d'un axe longitudinal de levier et être cependant bloquées.

6. Dispositif selon l'une des revendications 1 à 5,
**caractérisé en ce que** le dispositif d'actionnement (30) comprend un élément de calage (34) pouvant être déplacé suivant un mouvement rectiligne pour l'actionnement des éléments préhenseurs (21, 22) pivotants.

7. Dispositif selon la revendication 6,
**caractérisé en ce que** l'élément de calage (34) est couplé à une extrémité longitudinale d'un arbre d'actionnement (32) logé en translation longitudinale dans une section tubulaire (11) allongée du corps de base (11, 12, 16), lequel arbre, à son extrémité opposée, fait saillie de la section tubulaire (11) et supporte à ladite extrémité un élément d'actionnement manuel (31).

8. Dispositif selon l'une des revendications 1 à 7,
**caractérisé en ce que** ledit élément préhenseur (23) supplémentaire est monté fixe sur le corps de base (11, 12, 16).

9. Dispositif selon l'une des revendications 1 à 7,
**caractérisé en ce que** l'élément préhenseur (23) supplémentaire est maintenu de manière à pouvoir être déplacé selon un mouvement rectiligne et cependant bloqué sur le corps de base (11, 12, 16).

10. Système pour la saisie ou le suivi de la position d'un os (39), comprenant un dispositif selon l'une des revendications 1 à 9, un indicateur de position (40) monté dans le support (12) aménagé dans le corps de base (11, 12, 16), un détecteur (50) pour la saisie d'un signal émis par un indicateur de position (40), ainsi qu'une unité d'évaluation (60) branchée au détecteur (50), laquelle unité détecte à l'aide d'un signal de sortie du détecteur (50) et d'un cliché préalablement réalisé sur le patient un écart dans la position momentanée de l'os (39) par rapport à la position de l'os telle que représentée sur le cliché du patient.

11. Système selon la revendication 10, **caractérisé en ce qu'**une image du patient peut être générée à partir de l'unité d'évaluation (60) à l'aide du cliché préalablement réalisé sur le patient et du signal de sortie du détecteur, laquelle image montre la position momentanée de l'os (39), et **en ce qu'**une unité de visualisation (70) est prévue pour la visualisation de l'image ainsi générée du patient.
